Europäisches Patentamt

European Patent Office

Office européen des brevets

(19)

(11) Veröffentlichungsnummer : **0 396 638 B1**

## (12) EUROPÄISCHE PATENTSCHRIFT

(45) Veröffentlichungstag der Patentschrift :
**24.03.93 Patentblatt 93/12**

(51) Int. Cl.$^5$ : **A61K 35/78**

(21) Anmeldenummer : **89906713.6**

(22) Anmeldetag : **10.06.89**

(86) Internationale Anmeldenummer :
**PCT/DE89/00381**

(87) Internationale Veröffentlichungsnummer :
**WO 89/12454 28.12.89 Gazette 89/30**

(54) **HEILPFLANZENGEMISCH FÜR DIE BEHANDLUNG VON DEPRESSIONEN.**

(30) Priorität : **14.06.88 DE 3820218**

(43) Veröffentlichungstag der Anmeldung :
**14.11.90 Patentblatt 90/46**

(45) Bekanntmachung des Hinweises auf die
Patenterteilung :
**24.03.93 Patentblatt 93/12**

(84) Benannte Vertragsstaaten :
**AT BE FR GB IT LU NL SE**

(56) Entgegenhaltungen :
**Rote Liste, 1967, Editio Cantor (Aulendorf
Württ., DE) "Magentropfen Reichelon", S. 722
Chemical Abstracts, Band 94, Nr. 17, 27 April
1981 (Columbus, Ohio, US) A. Fundaro et al.:
"Interaction of chlorpromazine and the essential oils of absinthium and mace on operant
behavior in rats", page 73, abstract Nr
132339t.**

(73) Patentinhaber : **OMER, Osama L. M.
Zähringerstrasse 12
W-7888 Rheinfelden (DE)**

(72) Erfinder : **OMER, Osama L. M.
Zähringerstrasse 12
W-7888 Rheinfelden (DE)**

**Beschreibung**

Die Pflanzengemischzubereitung besitzt drei charakteristische Grundwirkungen:

1: beruhigend/angstlösend (sedativ/anxiolytisch)

2: stimmungsaufhellend (antidepressiv)

3: vegetativ stabilisierend

Die drei Wirkungen vereinigen sich zu einem Wirkspektrum das Komponenten von Tranquilizern, Antidepressiva und vegetativ regulierenden Mitteln umfaßt.

Die Wirkung des erfindungsgemäßen Pflanzengemischs erfolgt in zwei Phasen. In der ersten relativ rasch einsetzenden Phase (1. bis 5. Tag) lösen sich Angst und die Spannungen ohne Beeinträchtigung der körperlichen und geistigen Leistungsfähigkeiten. In der zweiten Phase (7. bis 14. Tag) kommt es zu einem Stimmungsumschwung, der von den Patienten als innere Befreiung erlebt wird. Die depressive Verstimmung klingt ab und die Patienten zeigen mehr Selbstvertrauen und Aktivität.

Zusammen mit der psychischen Harmonisierung geht die Stabilisierung des vegetativen Tonus. Dysregulationen des autonomen Nervensystems werden beseitigt und die funktionellen Organbeschwerden werden zum Abklingen gebracht.

Zielgruppen

Zielgruppe sind vor allem ängstliche, hypochondrische und deprimiert-verstimmte Patienten, bei denen die Störung des psychischen Befindens durch Schlaflosigkeit, Angst, innere Unruhe, Spannungen, nervöse Erschöpfung und durch "Minus-Symptomatik" zum Ausdruck kommt.

ANWENDUNGSGEBIETE

1. Störung des psychischen Befindens

1.1. Depressives Syndrom

1.1.1. Endogene Depressionen

1.1.2 Psychogene (neurotische) Depressionen

1.1.3. Somatogene Depressionen

1.2. Depressive Verstimmungen

1.3. Angstsyndrom

1.3.1. Somatische Angst (Organneurosen)

1.3.2. Phobien

1.3.3. Psychische (Pathologische) Angst

2. Vegetativ Funktionelle Syndrome

2.1. Kreislauf- und Atemfehlregulationen

2.2. Funktionelle Störungen im Magen-Darm-Bereich

2.3. Reizzustände der Harn- und Geschlechtsorgane

2.4. Endokrin bedingte funktionelle Störungen

2.4.1. Psychonervöse klimakterische Beschwerden

2.4.2 Neurovegetative klimakterische Beschwerden

2.4.3. Prämenstruelles Psychosyndrom

3. Migränen

4. Adjuvanstherapie bei psychoreaktiven Störungen bei

4.1. Angina Pectoris, Postinfarkt Psychosyndrom

4.2. Labile Hypertonie

4.3. Bronchialasthma

4.4. Psychogen bedingte Hautreaktionen

4.5. Chron. rez. Gastritiden und Reizkolon

Die einzelnen Bestandteile der erfindungsgemäßen Mischung werden seit Jahrhunderten in der euroasiatischen Volksmedizin angewendet und gelten für die menschliche Gesundheit als unbedenklich (siehe Bibliographie).

Keiner der genannten Bestandteile besitzt alleine die beanspruchte therapeutische Wirkung. Es wurde herausgefunden, daß auch eine beliebige Zweier- oder Dreier-Kombination der einzelnen Bestandteile nicht die oben beanspruchte therapeutische Wirkung zeigte. Eine Zubereitung aus Wermut, Rosenblüten, Cardamom und Mastixharz entfaltete eine gewisse Heilwirkung auf das psychische Wohlbefinden des Menschen. Mischt man dazu gepulvertes Bambus-Manna und/oder Agarholz bzw. Onosma-Blütenblätter , so tritt in nicht zu erwartender Weise die beanspruchte therapeutische Wirksamkeit zu Tage. Mit anderen Worten bedeutet dies, daß die erfindungsgemäße Mischung von Bambus-Manna und/oder Agarholz und/oder Onosma-blütenblätter zu der erfindungsgemäßen Mischung von Wermut, Rosenblüten, Cardamom und Mastixharz einen ganz ausgeprägten synergistischen Effekt mit sich bringt. Der Anteil der verschiedenen Pflanzen bzw. Pflanzenteile in dem erfindungsgemäßen Heilpflanzengemisch ist wie folgt:

10-70 Gew.-% Wermutkraut
01-30 Gew.-% Cardamomfrucht
10-60 Gew.-% Rosenblüten
05-40 Gew.-% Mastixharz
05-50 Gew.-% Agarholz
01-30 Gew.-% Bambus-Manna
10-60 Gew.-% Stores Onosma Brateatum
bezogen auf ein Gesamtgewicht aller Wirkstoffe von 100% .

Die Erfindung soll mit dem nachfolgenden Beispiel erläutert werden.

BEISPIEL

Die einzelnen unabhängig voneinander zu Feinpulver gemahlenen Ausgangsmaterialien werden miteinander gemischt, um das Stoffgemisch in homogener Form zu ergeben. Als besonders ausgewogen und optimiert zur Behandlung von Depressionen hat sich folgende Wirkstofzusammentsetzung der Arzneimittelzubereitung bewährt:

| Wermutkraut | (Artemisia absinthium) | 150 mg |
|---|---|---|
| Cardamomfrucht | (Fructus elettaria) | 30 mg |
| Rosenblüten | (Flores rosae damascena) | 90 mg |
| Mastixharz | (Resin pistacia lenticus) | 50 mg |
| Agarholz | (Aquilaria agallocha) | 60 mg |
| Bambus-Manna | (Resin bambusa arundinaceae) | 30 mg |
| Onosma Blüten | (Flores onosma brateatum) | 90 mg |

abgefüllt in Kapseln der Grösse OO.
Gesamtgewicht der Wirkstoffkombination 500 mg

Die nachfolgenden Versuche zeigen die Anwendbarkeit der Erfindung.

VERSUCH I:

THERAPEUTISCHER EINSATZ BEI DEPRESSIVEM SYNDROM

1. Wirknachweis mit Hilfe von Plazebo-kontrollierten Doppelblind-Studien.

Um die Wirksamkeit und Unbedenklichkeit des erfindungsgemäßen Pflanzengemischs bei der Behandlung von Depressionen zu prüfen, wurden 50 Patienten nach den DSM.III Kriterien (Diagnostic Statistical Manuel,1978. American Psychiatric Association) gewählt. Sie erfüllten außerdem noch die RDC (Research Diagnostic Criteria. Spitzer et. al. Arch. Gen. Psychiatry 35, 773, 1978.). Die klinische Prüfung wurde nach dem neuen AMG durchgeführt und die Einwilligung der Patienten eingeholt. Der Grad der Depression und die einzelnen Zielsymptome wurden mit Hilfe von Selbstauswertungsskalen, Visuell-Analog Skalen, Hamiltons Depressions Skala (HDRS) und Zungs Depressions Status Index (DSI) erfaßt. Die Dosis betrug 2x2 bis 3x3 Kapseln pro Tag (2 bis 4,5 g des erfindunsgemäßen Pflanzengemischs). Nach einem offenen "Singleblind Design" wurden die Patienten zunächst für drei Wochen mit dem erfindungsgemäßen Stoffgemisch behandelt. Nach dieser Zeit wurde die Behandlung randomisiert und doppelblind mit entweder Placebo (ähnlich aussehenden Kapseln) oder Verum für weitere 2 Wochen fortgesetzt. Verschlechterte sich während dieser 5-wöchigen Behandlung das klinische Bild oder blieb es unverändert während der ersten 2 Wochen, so wurden diese Patienten aus der Studie genommen. Patienten mit Suizidrisiko wurden von vornherein von der Studie ausgeschlossen.

Bereits in den ersten 5 bis 10 Tagen zeigte sich eine deutliche Verbesserung der depressiven Symptomatik bei 80% der Patienten. Bei weiteren 6% verbesserte sich der Zustand im Verlauf der weiteren 2. bis 3. Wochen. Am Ende der 1. Phase der Behandlung (3. Woche) fielen sowohl der mittlere HDRS-Wert als auch der DSI-Index auf Werte ab, die für eine gesunde Bevölkerung gelten. Bei mehr als 60% der Patienten, die in der 4. Woche auf Placebo umgestellt waren, verschlechterte sich die Erkrankung. Am Ende der Behandlung (5. Woche) bestand ein statistisch signifikanter Unterschied in den Mittelwerten von HDRS- und DSI-Skalen zwischen den beiden Behandlungsgruppen.

Aus den oben beschriebenen Ergebnissen kann gefolgert werden, daß das erfindungsgemäße Gemisch gute Wirksamkeit bei der Behandlung von depressivem Syndrom zeigt. Die Unbedenklichkeit kann aus den fehlenden Nebenwirkungen und den unveränderten Laborparametern geschlossen werden.

VERSUCH II:

THERAPEUTISCHER EINSATZ BEI DEPRESSIVEN VERSTIMMUNGEN

Der verstimmte Patient ist im Gegensatz zum depressiven Patienten emotionell durchaus noch beweglich. Diese Form der Depression trifft man am häufigsten in der ärztlichen Sprechstunde. Die Verstimmungszustände treten oft gemeinsam mit Angstzuständen auf. Um die Wirksamkeit und Unbedenktichkeit des erfindungsgemäßen Stoffgemischs bei der Behandlung von depressiven Verstimmungen zu prüfen, wurden 160 Patienten mit dem erfindungsgemäßen Stoffgemisch (2 bis 3 g täglich) 4 Wochen lang behandelt.

Alle Patienten mußten, um in die Studie aufgenommen zu werden, die DSM-III Kriterien von "Major Depressive Disorder" (Diagnostic Statistical Manual, American Psychiatric Association 1978) erfüllen. Die Studie wurde in der Praxis des Autors nach den Richtlinien des neuen AMG durchgeführt. Der Schweregrad der Verstimmung wurde mit Hilfe der Selbstauswertungsskalen sowie mit Hilfe von Hamiltons Depressionsskala (HDRS) ermittelt. Eine deutliche Besserung zeigte sich bereits in der ersten Behandlungswoche. Nach 4 Wochen waren es weniger als 10% der Patienten, die keine oder nur eine mäßige Wirkung zeigten. Die Ergebnisse belegen wiederum die gute Wirkung des Gemischs bei der Behandlung von depressiven Verstimmungszuständen.

VERSUCH III

THERAPEUTISCHER EINSATZ BEI DER VEGETATIVEN UND VEGETATIV-DYSTONE DEPRESSION

Depressionen, deren Erscheinungsbild durch das Vorherrschen einer körperlichen, vor allem vegetativen Symptomatik gekennzeichnet ist und bei denen die psychischen Phänomene in die Nebenrolle gedrängt sind, werden vegetative, vegetativ-dystone oder auch somatisierte Depressionen genannt. Die depressiven Zustandsbilder werden bei solchen Patienten häufig nicht diagnostiziert, weil die Kranken von der Macht der somatischen Störungen so überwältigt sind, daß sie beim Arzt ausschließlich über körperliche Beschwerden klagen.

Um eine depressive Symptomatik hinter der körperlichen Maske aufzudecken, wurden dem Patienten im Laufe des Gesprächs folgende gezielte Eragen gestellt:

1. Wie geht es mit Ihren Interessen? Sind Sie in letzter Zeit lustlos oder interessenlos? (INTERESSENLOSIGKEIT)

2. Fühlen Sie sich tagsüber erschöpft, ohne Schwung? (MÜDIGKEIT, ENERGIELOSIGKEIT)

3. Fühlen Sie sich nervös, innerlich gespannt, unruhig oder ängstlich? (RUHELOSIGKEIT)

4. Haben Sie in der letzten Zeit zu wenig Appetit und Gewicht abgenommen oder zu viel Appetit bzw. ungewollt zugenommen? (APPETITSTÖRUNGEN).

5. Fällt es Ihnen schwer, in der letzten Zeit Entscheidungen zu treffen oder sich zu konzentrieren? (KONZENTRATIONSTÖRUNGEN)

6. Haben Sie ungerechtfertigte Schuldgefühle oder grübeln Sie zu viel? (SCHULDGEFÜHL)

7. Ist Ihr Schlaf gestört oder schlafen Sie zu viel? (SCHLAFSTÖRUNGEN)

8. Plagt Sie das Gefühl, Ihr Leben sei sinnlos geworden? Haben Sie manchmal Lebensüberdruß oder Selbstmord-Gedanken? (LEBENSÜBERDRUSS)

Nach den DSM.III Kriterien von American Psychiatric Association für "Major Depressiv Episode" genügt es, wenn vier der oben gennanten acht Eragen mit "Ja" beantwortet werden, um die-Diagnose "Depression" zu stellen.

Die Wirksamkeit und Unbedenklichkeit des erfindungsgemäßen Stoffgemischs bei vegetativen bzw. psychovegetativ-dystonischen, somatisierten, maskierten, oder larvierten Depressionen wurde durch folgende kontrollierte und offene Studien untermauert.

a) "Single-Blind" Studie vs. Bromazepam

Vierzig vegetativ-dystonisch depressive Patienten erhielten über 3 Wochen im Rahmen einer "Single-Blind" Studie 1 bis 4 mg Bromazepam (Lexotanil) oder 1 bis 4 Kapseln, die je 500 mg des erfindungsgemäßen Stoffgemischs enthielten. Neben dem Verstimmungsgrad, der mit Hilfe der HDRS-Skala erfaßt wurde, wurden die einzelnen körperlichen Störungen als Zielsymptome mit Hilfe von Visual-Analog Selbstauswertungs-Skalen erfaßt.
Das erfindungsgemäße Stoffgemisch zeigte - verglichen mit Bromazepam - in der 3 Woche der Auswertung eine deutliche, statistisch signifikante Überlegenheit.

b) Offene Erfahrungsstudien

Sechzig vegetativ-dystonisch depressive Patienten erhielten über vier Wochen 2 bis 4 g des erfindungsgemäßen Stoffgemischs. Neben dem Verstimmungsgrad, der mit Hilfe der HDRS-Skala erfaßt wurde, wurden einzelne körperliche Störungen als Zielsymptome mit Hilfe von Visual-Analog Selbstauswertungsskalen erfaßt. Das erfindungsgemäße Stoffgemisch konnte 52 Patienten beschwerdefrei machen.

VERSUCH IV

THERAPEUTISCHER EINSATZ BEI ANGSTZUSTÄNDEN

Die pathologische Angst, eine sehr häufige Erscheinung, wird sehr oft mit sedativen Anxiolytica - besonders mit Benzodiazepinen - behandelt. Starke Dosissteigerungen werden vom Arzt oft geduldet und die Verschreibung erstreckt sich auf Wunsch der Patienten über lange, manchmal über unbegrenzte Zeitspannen, so daß iatrogene Drogenabhängigkeit und Drogensucht zu einem Problem geworden ist. Um zu prüfen, ob das erfindungsgemäße Stoffgemisch bei der Bekämpfung von pathologischen Angst-Reaktionen, insbesondere mit vorherrschender somatischer Symptomatik und überschießender Streßreaktion mit Erfolg eingesetzt werden kann und ob das Gemisch die Nachteile von den Benzodiazepinen nicht aufweist, wurden die Behandlungsresultate von 70 Patienten aus der eigenen Privatpraxis ausgewertet.

Offene Studien an Angst-Patienten.

Ausschlaggebend für die Ausnahme eines Patienten in die Studie war die Diagnose "pathologische Angst". Das untersuchte Krankengut wurde in vier symptomorientierte Untergruppen eingeteilt.
Gruppe I: Diese Gruppe umfaßte die Patienten, bei denen sich die pathologische Angst in erster Linie auf psychischer Ebene äußerte, sei es in Form eines permanenten Zustandes, sei es anfallsweise, vorausgesetzt, daß die Anfälle durch keinen besonderen Anlaß ausgelöst wurden und psychische Manifestationen der Angst vorherrschten. Diese Gruppe bestand aus 20 Patienten (Siehe Tabelle 1).
Gruppe II: Diese Gruppe bestand aus 15 Patienten mit phobischer Angst, deren vorwiegend in psychischen oder somatischen Symptomen sich äußernde Angstexazerbationen durch bestimmte Stimuli ausgelöst zu sein schienen.
Gruppe III: Diese Gruppe umfaßte 20 Patienten, bei denen die Angst mit gedrückter Stimmung einherging, oft mit Verlustgefühlen und mit einer Beeintächtigung der Konzentrationsfähigkeit sowie mit Antriebsschwäche und Energiemangel.
Gruppe IV: Diese Gruppe aus 25 Patienten drückten Angst vorwiegend in somatischen Symptomen aus, die entweder allgemeiner oder mehr kardiovaskulärer, respiratorischer, gastrointestinaler, urogenitaler bzw. vegetativer Natur waren.
Der Schweregrad der Angst wurde mit Hilfe der 14 Punkte umfassenden Angstskala von Hamilton ermittelt. Die Gesamtzahl für die Punkte 1 bis 6 und des Punktes 14 wurde der Gesamtpunktzahl für die Items 7 bis 13 gegenübergestellt. Nach Korrektur mit einem konstanten Gewichtungsfaktor wurden die Patienten je nach der Symptomart, welche die höhere Summe erreichte, der "psychischen" oder der "somatischen" Gruppe zugeteilt. Die Bewertung mit Hamiltons Angstskala erfolgte vor Beginn der Behandlung und in den ersten vier Wochen der Studie in wöchentlichen Abständen sowie nach Abschluß der Therapie und wurde spätestens nach drei Monaten wiederholt. Keiner der Patienten erhielt während der Dauer der Studie zusätzliche Psychopharmaka, allerdings wurde die vorherige somatische Dauerbehandlung als Zusatzbehandlung fortgesetzt. Die Dosierung bewegte sich je nach Ansprechen des Patienten zwischen 3 bis 8 Kapseln pro Tag. Verschlechterte sich der Zustand des Patienten während der Behandlung mit dem erfindungsgemäßen Stoffgemisch oder trat keine nennenswerte Verbesserung während der ersten drei Wochen der Behandlung ein, so wurde der Patient aus der Studie genommen. Die Ergebnisse sind in der Tabelle 1 zusammengefaßt.

TABELLE    1: Behandlung von Angstzuständen

|  | Gruppe I | Gruppe II | Gruppe III | Gruppe IV |
|---|---|---|---|---|
| Fallzahl | 20 | 15 | 20 | 25 |
| Erfolglos | 5 | 6 | 7 | 8 |

Aus der hier besprochenen Studie geht eindeutig hervor, daß das erfindungsgemäße Stoffgemisch eine sinvolle und nützliche Kombination für die Behandlung von verschiedenen Angstzuständen darstellt. Bei etwa zwei Drittel der Patienten wurde innerhalb eines kurzen Zeitraums eine befriedigende klinische Besserung erzielt - ein Resultat, das den Vergleich mit Ergebnissen der Benzodiazepin-Therapie aushält. Das Ausmaß der Besserung äußerte sich in der Abnahme des mit der Hamilton-Skala berechneten mittleren Gesamtwertes der Angst auf ein Drittel des Ausgangwertes vor der Behandlung und in der Selbstbeurteilung durch die Patienten. Nebenwirkungen wurden nicht beobachtet. Patienten mit somatischen Angstsymptomen sprachen besser auf die Therapie an als Patienten mit Phobien. Aber auch in der Kategorie Angst mit begleitender Depression gab es in über zwei Drittel der Fälle eine deutliche Besserung. Mit diesem Ansprechen in den einzelnen Gruppen unterscheidet sich das Stoffgemisch von den Benzodiazepinen. Der Unterschied zu Benzodiazepinen im vorliegenden Zusammenhang kam am deutlichsten bei den Patienten zum Ausdruck, bei denen die Medikation über drei Monate oder länger fortgesetzt werden mußte. Nach Beendigung der Therapie kam es bei keinem Patienten zu Erscheinungen, die auf Entzugssymptomatik schließen lassen konnten.

VERSUCH IV

SCHLAFSTÖRUNGEN

Schlafstörungen gehören zu den häufigsten funktionellen Störsymptomen. Sechzig (60) Patienten mit Schlafstörungen und Verstimmungen erhielten zwei Wochen lang zwecks Schlafinduktion 4 Kapseln (bei Bedarf abends 2 Kapseln zusätzlich) der erfindungsgemässen Zubereitung.
Die Selbstbeurteilung der Patienten ergab eine schlafanstoßende Wirkung bei 85% der Patienten.
Nach zwei Wochen der Behandlung wurden die Kapseln, die die erfindungsgäße Zubereitung enthielten, für eine Woche durch ähnlich aussehenden Plazebo Kapseln in der gleichen Anzahl pro Tag ersetzt. Die Schlafqualität verschlechterte sich erneut bei mehr als der Hälfte der Patienten am Ende dieser Plazebo-Woche. Die Einschlafzeit betrug vor Beginn der Behandlung im Durchschnitt über eine Stunde, am 5. Behandlungstag nur noch ca. 15 Minuten und nach zwei Wochen ca. 10 Minuten. Am Ende der Plazebo-Woche erhöhte sich die Einschlafzeit erneut auf über eine halbe Stunde.
Die allgemeine Verträglichkeit war gut. Die Tagesmüdigkeit nahm mit fortschreitender Normalisierung des Schlafes ab.
Die oben beschriebene kontrollierte Studie belegt eindeutig die gute schlafanstoßende Wirkung.
Zusammenfassung siehe Tabelle 2.

Tabelle 2:    Schlafqualität

|  | Baseline | 7. Tag | 14. Tag | 7. Tag |
|---|---|---|---|---|
|  |  |  |  | Placebo |
| Sehr gut | 0 | 25 | 30 | 8 |
| Gut | 0 | 5 | 2 | 10 |
| Leicht gestört | 11 | 8 | 4 | 10 |
| Stark gestört | 29 | 2 | 6 | 12 |

BESCHREIBUNG DER EINZELNEN BESTANDSTEILE

Die einzelnen Bestandteile der erfindungsgemäßen Mischung sind seit vielen Jahrhunderten für die menschliche Gesundheit nützlich und als nicht toxisch bekannt. Sie sind in der Literatur sehr ausführlich beschrieben worden. (Siehe Bibliographie). Im folgenden sollen die Ausgangsstoffe des erfindungsgäßen Stoffgemischs nur kurz erläutert werden.

WERMUKRAUT

Handelsbezeichnung:

Herba absinthii vulgaris, Wermutkraut, Absinth, Wurmkraut, Bitterer Beifuß, Wormwood.

Bestandteile

Das trockene Kraut enthält 0,25-1,32% ätherisches Öl, Absinthin, Anabsin, Anabsinthin , Artabsin und Matricin. Das ätherische Öl enthält Thujon und Thujylalkohol sowie eine Reihe weiterer Terpen-Derivate. Eine gute Handelsdroge soll einen Absinthingehalt von mindestens 0,2%, (spektralphotometrisch bestimmt) haben, was einem Bitterwert von ca. 15000 entspricht.

Verwendungen

Verwendet wird das Kraut unter anderem als Appetitanreger, als Wurmmittel und als Wermutwein. Hervorzuheben ist der karminative, choleretische Effekt, der auf die Bedeutung der Droge als Gallenmittel hinweist.

CARDAMOM-FRUCHT

Handelsbezeichnung:

Amomum verum, Cardamomum rotundum, Cardamomum racemosu, Kapol, Siam Cardamom, Camphor seeds.
Verwendung: Im indischen Subkontinent und in Südostasien in der Anwendung als Gewürz weit verbreitet. Inhaltsstoffe: ca. 2,4% ätherische Öle, Fettöle, Stärke und Kohlenhydrate.

ROSENBLÜTEN

Handelsbezeichnung:

Flores Rosae rubrae, petala Rosae, ebenfalls die Kulturform Rosa Gallica, Rosa Centifolia und triginitipetala.

Inhaltsstoffe:

10-24% Gerbstoffe, ca 0,01% Äther. Öl (Flavonglycosid), Cyanin und Aglykone. Distillationsöle enthalten 40-76% Geraniol, 5-10% Nerol, 15-37% l-Citronellol, Citronellal, Citral, ca 1% Eugenol, 1-Linaool, Farnesol, ca 3-5% Ester der obigen Alkohole, 15-30% Stearoptene.

Verwendung:

Die Rosenblüten finden als Astringens, bei Durchfall, in der Parfümindustrie und als Bestandteil zahlreicher Pharmapräparate Anwendung.
Eine cholagoge, antiphlogistische sowie eine antibakterielle Wirkung des Rosenöls konnte festgestellt werden.

MASTIXHARZ

Handelsbezeichnung:

Mastiche, Mastix, Gum of Mastic. Rumi-Mastiki

Inhaltssoffe:

ca. 30% alpha-Masticoresen, ca. 20% beta-Masticoresen, ca. 20% alpha-Masticonsäure, ca. 18% beta-Masticonsäure, ca 4% alpha- und beta-Masticinsäure, ca. 0,58 Masticol-säure, ca. 2% Äther. Öle mit Pinen und Bitterstoff.

Verwendung:

Als Kaumittel, als Zusatz zu Mundwässer, Zahntinkturen, zu Pflastern und Pillen und in Lösungen zu Wundverbänden.In der Likörindustrie zur Harzung. Bestandteil von Soda-Getränken. Fixateur in der Parfümindustrie.
In der indischen Volksmedizin als Cholagogum und als Astringent, bei Impotenz, bei Verstopfungen und Lebererkrankungen.

AGARHOLZ

Handelsbezeichnung:

Agaru, Hindiagara, Aquilaria agallocha, Ud-Hindi.
Inhaltstoffe: Ätherische Öle

Verwendung:

Getrocknetes, pulverisiertes Holz findet Verwendung in der indischen Volksmedizin als Carminativum, Tonic, Astringent bei Durchfall und Erbrechen, als mildes Stimulans und in der Parfümindustrie.

BAMBUS-MANNA

Handelsbezeichnung:

Tabasheer, Tabashira, Siliciousconcentration of the milky bark of bamboo. Resin bambusa arundiacea.
Inhaltsstoffe: 90 % Silicium-Hydrat, Peroxid von Eisen, Kalium und Aluminium, Cholin, Betain, Fermente wie Nuclease, Urease, Diastase und sonstige protolytische und emulgierende Fermente.

Verwendung:

Antispasmodisch, Aphrodisiaka, Astrigent, Tonikum für das Herz und die Leber, bei Erbrechen, Palpitationen, Irritationen des Körpers, mildes Sedativum, bei Gelbsucht und verstärktem Durst.

ONOSMA BLÜTENBLÄTTER

Handelsbezeichnungen:

Guligaozabana, Kazabun, flowers of Gaozaban, Shankhahuti.

Verwendung:

In der indischen Volksmedizin als Tonikum, bei rheumatischen Erkrankungen, Lues, Lepra, Hypochondriasis, Nieren- und Leber-Erkrankungen. Bei Fieber zum Durststillen, bei funktionellen Störungen von Herz und Blase.

BIBLIOGRAPHIE

1. Drogenkunde. Heinz A. Hoppe, de Gruyter Verlag.
2. Useful Plants of India and Pakistan. Dastur . E.; Tarapurevala and Sons. 210 Hornby Road, Bombay, India.
3. Indian Materia Medica. Ed.: Nadkarni A. K.; Poular Book Depot, Bombay India, and Dhootapapeshwer, Parkashan Ltd. Panvel, Bombay, India.
4. Glossary of Indian Medicinal Plants. Council of Scientific and Industrial Research, India.
5. Hagers Handbuch der Pharmazeutischen Praxis. Ed.:P.H.List, L. Hörhammer. Springer Verlag Berlin, Heidelberg, New York.
6. AB-DDR, Arzneibuch der DDR Dtsch. Akademie Verlag, Berlin.
7. Handbuch der Drogenkunde. Ed.: F. Berger Verlag W.Maudrich, Wien.
8. Pharmazeutische Biologie. Ed.:F. Deutschmann, B. Hohmann E. Sprecher, E. Stahl. G. Fischer Verlag, Stuttgart, New York.
9. Arznei- und Gewürzpflanzen, Ein Leitfaden für Anbau und Sammlung. Ed.:K. Ebert. Wissenschaft Verlagsges., Stuttgart.
10. Handbuch des Arznei- und Gewürzpflanzenbaus. Deutscher Bauernverlag, Berlin.
11. Illustrierte Flora von Mitteleuropa. Verlag Paul Parey, Berlin, New York.

**Patentansprüche**

1. Heilpflanzengemisch als Pulver oder Extrakt, in Verbindung mit üblichen Applikationsträgern für die entsprechenden Arzneimittelzubereitungen, zur Behandlung von Depressivem Syndrom mit angstlösender, stimmungsaufhellender und vegetativ regulierender Wirkung, gekennzeichnet durch folgende Pflanzen bzw.
Pflanzenteile:
10-70 Gew.-% Wermutkraut
01-30 Gew.-% Cardamomfrucht
10-60 Gew.-% Rosenblüten
05-40 Gew.-% Mastixharz

2. Heilpflanzengemisch als Pulver oder Extrakt, in Verbindung mit üblichen Applikationsträgern für die entsprechenden Arzneimittelzubereitungen, zur Behandlung von Depressivem Syndrom mit angstlösender, stimmungsaufhellender und vegetativ regulierender Wirkung, gekennzeichnet durch folgende Pflanzen bzw.
Pflanzenteile:
10-70 Gew.-% Wermutkraut
01-30 Gew.-% Cardamomfrucht
10-60 Gew.-% Rosenblüten
05-40 Gew.-% Mastixharz
wenn dazu 05-50 Gew.-% Agarholz oder/und 01-30 Gew.-% Bambus-Manna oder/und 10-60 Gew.% Onosma-Blüten zugegeben und das Gesamtgewicht aller Wirkstoffe auf 100% bezogen wird.

**Claims**

1.  A combination of medicinal plants, mixed as dried powder or as an extract, in association with the usual pharmaceutical carrier substances of medicinal preparations, for the treatment of depressive syndrom with antianxiety, mood alleviating and vegetative regulatory properties, consisiting of folowing plants or part of the plants:
    10-70 weight % wormwood
    01-30 weight % seeds of cardamom
    10-60 weight % buds of roses
    05-40 weight % resin of mastiche

2.  A combination of medicinal plants, mixed as dried powder or as an extract, in association with the usual pharmaceutical carrier substances of medicinal preparations, for the treatment of depressive syndrom with antianxiety, mood alleviating and vegetative regulatory properties, consisiting of folowing plants or part of the plants:
    10-70 weight % wormwood
    01-30 weight % seeds of cardamom
    10-60 weight % buds of roses
    05-40 weight % resin of mastiche
    when 05-50 weight % wood of agar plant and/or 01-30 weight % pith of bamboos and/or 10-60 weight % flowers of gaozuban are added.

**Revendications**

1.  Mélange de plantes médicinales en poudre ou en extrait, combiné avec des supports classiques appropriés pour les préparations de médicaments, pour le traitement du syndrome de la dépression, avec effets de faire disparaître la peur et la dépression et de réguler le système neurovégétatif, caractérisé par les plantes, ou plutôt les parties de plantes suivantes:
    10-70 poids % herbes d'absinthe
    01-30 poids % fruit de cardamom
    10-60 poids % pétales de rose
    05-40 poids % résine de mastic

2.  Mélange de plantes médicinales en poudre ou en extrait, combiné avec des supports classiques appropriés pour les préparations de médicaments, pour le traitement du syndrome de la'dépression, avec effets de faire disparaître la peur et la dépression et de réguler le système neurovégétatif, caractérisé par les plantes, ou plutôt les parties de plantes suivantes:
    10-70 poids % herbes d'absinthe
    10-30 poids % fruit de cardamom
    10-60 poids % pétales de rose
    05-40 poids % résine de mastic
    si on ajoute 05-50% ou/et bois d'agar ou/et 01-30 poids % manna de bambus ou/et 01-60 flores onsma brateatum